# EUROPEAN PATENT APPLICATION

(11) **EP 1 352 960 A1**
(43) Date of publication of application: **15.10.2003**
(21) Application number: 02076434.6
(22) Date of filing: 12.04.2002
(51) Int. Cl.: C12N 15/11, C12N 5/10, A61K 48/00

(54) **Antiviral therapy on the basis of RNA interference**

(71) Applicant: Viruvation B.V., 2333 AL Leiden (NL)
(72) Inventor: de Haan, Petrus Theodorus, 2343 RR Oegstgeest (NL)
(74) Representative: Prins, Adrianus Willem

(57) **Abstract**

The invention concerns gene therapy for treatment of animals and humans which suffer from an infection with a chronic virus such as HIV or HCV. It can also be used prophylacticly to prevent chronic infection. The therapy makes use of a nucleotide construct which is able to produce a single transcript or multiple transcripts capable of folding into double-stranded RNA, which inhibits replication of the virus *in situ*.

## Description

The invention relates to a therapy against chronic viruses, such as HIV and *Hepatitis* viruses. In particular the invention relates to a new gene-technology based therapy to inhibit virus replication and to prevent the formation of new virus particles.

Chronic viruses are viruses with long latency periods in infected individuals, before disease symptoms develop. Chronic viruses include human immunodeficiency virus (HIV), Karposi's sarcoma-associated herpesvirus (KSHV), Eppstein-barr virus (EBV), *Hepatitis* B virus (HBV) and *Hepatitus C* virus (HCV). Especially HIV and HCV are a major global health concern. Patients infected with HIV will at a certain point in time lose immune responses and the typical symptoms of the lethal acquired immune deficiency syndrome (AIDS) will become visible. HCV is the causal agent of chronic liver infections, which develop into lethal cirrhosis or liver cancer in a substantial percentage of infected individuals. To date patients which carry these chronic viruses cannot be cured. The current anti-HIV therapies are based on the use of protease and reverse transcriptase inhibitors to prevent the formation of new virus particles (HAART). However, HAART is expensive and therefore not affordable by those countries that are the most affected. The inhibitors have many negative health effects and patients have to use the medication lifelong. HCV infections are currently treated with alpha-interferon alone or in combination with ribavirin, albeit with a low success rate. Additionally, the costs of both drugs are very high.

Major part of the research is therefore dedicated to the development of vaccines. However, due to the high mutation rates of the HIV and HCV genomes and hence the rapid emergence of virus variants, which escape from the above mentioned therapies, novel therapies are urgently needed.

HIV belongs to the lentivirus sub-family within the *Retroviridae.* Virus particles consist of two genomic single stranded RNA molecules wrapped up with protein to nucleocapsids. Lipid envelopes wearing glycoprotein projections surround the nucleocapsids. Virions enter specific lymphoid cells by binding of gp120 to CD4 receptors. The maturational stages of all hemopoietic cells are distinguished by their expression of membrane molecules recognised by specific monoclonal antibodies (clusters of differentiation, CD's). Hence, the T helper cells decorated with CD4 receptors are the main targets. However a number of other cell types such as monocytes, macrophages, dendritic cells, certain B-cells and hemopoietic stem cells (HSC's) also express some CD4 and as a consequence can be infected with HIV. Upon entrance the nucleocapsids are released in the cytoplasm where the reverse transcriptase (included in the nucleocapsids) produces a cDNA copy of the genomic RNA. The reverse transcriptase and RNAseH, also included in the nucleocapsids produce linear double stranded DNA copies of the genomic RNA, which are targeted to the nucleus where they are integrated as proviral DNA into the nuclear DNA by a viral integrase, which is also included in the nucleocapsids.

The proviral genomes are flanked by long terminal repeats (LTR's). The 5' LTR contains enhancer and promoter sequences essential for transcription of viral genes. The 3' LTR contains the polyadenylation signals, required for polyadenylation of the viral transcripts. The provirus contains three structural genes: gag; encoding the nucleocapsid proteins, pol; encoding the viral enzymes, and env; encoding the membrane glycoproteins. The mature proteins are formed after proteolytic cleavage of the primary translation products. In addition, the provirus has another five genes encoding regulatory proteins involved in virion infectivity, viral assembly & release, viral mRNA translocation and genome activation. The provirus is activated by antigen binding or by infection with other viruses. Provirus activation leads to expression of the regulatory proteins, including tat and rev. Tat will further strengthen the 5' LTR promoter, whereas rev will promote expression of the structural proteins and the viral enzymes. Viral nucleocapsids are formed in the cytoplasm and finally, virus particles will bud from the virus-infected CD4 cells. In this process, the virus however only kills T helper cells, which have higher than threshold CD4 receptor levels. The other infected cell types remain in tact.

HIV infections therefore will at a certain point in time lead to a depletion of CD4+ T-cells and the typical AIDS symptoms will become visible. Uninfected CD4 T-cells lose their capacity to respond to new antigens. General antibody levels will decline. T-cell maturation in the thymus will be inhibited. Cytokine imbalances will occur leading to many immunological abnormalities. AIDS patients will finally die from progressive pathogen infections and exhaustion.

HCV belongs to the *Flaviviridae.* Virus particles consist of a plus stranded genomic RNA molecule wrapped up with C protein to nucleocapsids. Lipid envelopes with glycoprotein projections surround the nucelocapsids. The RNA molecule encodes a single large polyprotein, which is co- and post-translationally cleaved by viral and cellular proteases into 10 different mature viral proteins, with the structural proteins (C, E1, E2 and p7) located in the amino-terminal part and the non-structural proteins (NS2, NS3, NS4a, NS4b, NS5a and NS5b) located at the carboxy terminus. Virus particles have a tropism for hepatocytes and lymphoid cells. Upon entrance, the nucleocapsids are released in the cytoplasm and the genomic RNA molecules are translated directly. Virus replication takes place in the cytoplasm of infected cells. The majority of infected individuals becomes chronically infected and frequently shows chronic *Hepatitis.* The history of chronic HCV infections can vary dramatically between individuals. Some have minimal liver disease and never develop complications. Others develop severe and often lethal cirrhosis and/or hepatocellular carcinoma's after many years of infection. Cirrhosis is momentarily the leading indication for liver transplantations. HCV is characterised by its presence at very low titres in the infected cells during the chronic phase and by its extremely high genomic variability. These characteristics significantly contribute to the lack of successful anti-HCV therapies.

Gene therapy has been considered as an attractive alternative novel anti-AIDS therapy (Romano et al., Stem Cells 17: 191-202, 1999; Engel & Kohn, Front. Biosci. 4: 26-33, 1999; Buchsacher & Wong-Staal, Human Gene Therapy 12: 1013-1019, 2001). To date, gene therapy against diseases caused by other chronic viruses has not been suggested.

There are two general approaches to gene therapy for HIV infection: to eliminate infected cells or to interfere with viral replication. The first approach includes attempts to increase the immune response in a patient directed against HIV and attempts to express toxins in HIV-infected cells. The second approach reported so far includes transgenic expression of dominant negative viral proteins (US 5,908,923); targeted nucleases & ribonucleases (Singwi & Joshi, Front. Biosci. 5: 556-579, 2001; Singwi *et al*., Gene Therapy 6: 913-921, 1999); intracellular antibodies (Marasco *et al*., Human Gene Therapy 9: 1627-1642, 1998; Mhasshilkar *et al*., Human Gene Therapy 10: 1453-1467, 1999); interfering RNA molecules (Lamothe & Joshi, Front. Biosci. 5: 527-555, 2000) such as sense decoy RNA molecules (Rosenzweig et al., J. of Virol. 71: 2740-2746, 1997; Kohn et al., Blood 94: 368-371, 1999), antisense RNA molecules (WO 94/16066; DE 4225094; EP 0 386563; Veres et al., J. of Virology 72: 1894-1901, 1998) and HIV-specific ribozymes (WO 94/26877; Rigden et al., Curr. Issues Mol. Biol. 2: 61-69, 2000). To date only in vitro experiments have been performed using (HIV-infected) human cell lines or CD34+38- cells isolated from patient's blood samples.

Comparable approaches have been used to confer virus resistance in other living systems such as plants. In plants, ribozymes, antisense & sense decoy RNA's confer virus-resistance at relatively low levels and/or inefficiently e.g. only a low percentage of the transformed cells (plants) show the desired phenotype. From these experiments it can be deduced that the levels of inhibition of HIV replication conferred by these molecules is too low to fully keep HIV proviral DNA molecules in a latent state.

Thus there is still need for a more robust and efficient gene therapy for the inhibition of the replication of HIV and other chronic viruses including HCV.

### Summary of the invention

The invention now provides a method for the inhibition of replication of a chronic virus in cells of an animal or human by introducing into target cells of the virus a nucleotide construct which is able to produce one transcript or multiple transcripts capable to fold into a double stranded RNA (dsRNA) with nucleotide sequence homology to one or more nucleotide sequences of the virus which are essential for replication of the virus. Alternatively, the invention provides such a method wherein a stem cell is provided with the nucleotide construct and wherein said stem cell is capable of generating the target cell of the virus.

In the specific case of HIV the invention provides for a method for inhibition of HIV replication comprising
a. isolating hemopoietic or lymphoid stem cells from a person;
b. introducing into these stem cells a nucleotide construct which is able to produce one transcript or multiple transcripts capable to fold into a dsRNA with nucleotide sequence homology to one or more nucleotide sequences of the virus which are essential for replication; and
c. re-introducing the stem cells in the person from whom they have been isolated.

Further, the invention comprises an animal or human cell which is the target cell for a chronic virus, characterized in that it is transformed or transfected with a nucleotide construct which is able to produce one transcript or multiple transcripts capable to fold into a dsRNA with nucleotide sequence homology to one or more nucleotide sequences of the virus which are essential for replication,. In the alternative an animal or human stem cell is provided which is able to generate a target cell for a chronic virus, characterized in that it is transformed or transfected with a nucleotide construct which is able to produce one transcript or multiple transcripts capable to fold into a dsRNA with nucleotide sequence homology to one or more nucleotide sequences of the virus which are essential for replication,.

More specifically such a cell is a human hemopoietic or lymphoid stem cell, characterized in that it is transformed or transfected with a nucleotide construct which is able to produce one transcript or multiple transcripts capable to fold into a dsRNA with nucleotide sequence homology to one or more nucleotide sequences of the human immunodeficiency virus which are essential for replication.

Also part of the invention is a nucleotide construct harbouring a nucleotide sequence of at least 40 nucleotides, which shares nucleotide sequence homology to a gene or part of a gene of a chronic virus which is able to infect an animal or human, wherein said nucleotide sequence is also present as an inverted repeat or wherein said nucleotide sequence is flanked by two promoters and which construct, when transcribed yields one transcript or multiple transcripts capable of folding into a dsRNA from said sequence and its inverted repeat or from said sequence flanked by two promoters. Preferably in such a nucleotide construct the nucleotide sequence and its inverted repeat are separated by an intron. Another embodiment of the invention is such a nucleotide construct which comprises multiple nucleotide sequences of at least 40 nucleotides long which are homologous to different parts of the same gene or to different genes or parts thereof of said virus, wherein said nucleotide construct is also present as an inverted repeat, or wherein said nucleotide construct is flanked by two promoters.

### Detailed description of the invention

Inhibition of replication of a chronic virus is obtained by a method of producing sequence-specific inhibition of gene expression by introducing a nucleotide construct capable of forming upon transcription one transcript or multiple transcripts, capable of folding into dsRNA. A process is provided for inhibition of expression of a homologous viral gene in a human or animal cell, which is the target for the virus or which can generate cells which are the target cells for the virus. The process comprises introduction of nucleotide constructs comprising nucleotide sequences homologous to sequences of the virus which are essential for replication which may fold into partially or fully dsRNA molecules. When present in the target cells such nucleotide constructs inhibit expression of the homologous viral gene(s) in a sequence-specific manner, in a process referred to as RNA silencing (Ding S.W. 2000, Curr. Opin. Biotechnol. 11: 152-156). Inhibition of gene expression refers to the absence (or observable decrease) in the level of protein and/or mRNA product form the homologous viral gene(s). Specificity refers to the ability to inhibit the target gene without manifest effects on other genes of the cell.

Alternatively, the cell may be directly provided with a dsRNA molecule.

A nucleotide construct which is able to form RNA containing a nucleotide sequence identical to a portion of the viral gene is preferred for inhibition. RNA sequences with insertions, deletions and single point mutations relative to the target viral sequence (i.e. not identical but homologous to the viral sequence) have also been found effective for inhibition. This is especially valuable in circumstances where the virus is prone to genomic variation. Even if the virus' genomic sequence shows variation the inhibiting effect of the nucleotide construct pertains. Anyway, sequence identity may be optimised by sequence comparison and alignment algorithms known in the art (see Gribskov and Devereux, Sequence *Analysis Primer,* Stockton Press, 1991, and references cited therein), and calculating the percent difference between the nucleotide sequences, for instance by using the Smith-Waterman algorithm as implemented in the BESTFIT software program using default parameters (e.g. University of Wisconsin Computing Group). Greater than 90% sequence identity, or even 100% sequence identity, between the inhibitory nucleotide sequence and the portion of the target viral gene is preferred. Alternatively, the duplex region of the RNA transcript or transcripts encoded by the nucleotide construct may be defined functionally as a (double stranded) nucleotide sequence that is capable of hybridising with a portion of the target gene transcript (e.g., 400 mM NaCl, 40 mM PIPES pH 6.4, 1 mM EDTA, 50°C to 65°C hybridisation for 12-16 hours; followed by washing). The length of the identical nucleotide sequences should be at least 40 nucleotides, but preferably larger: 50, 100, 200, 300 or 400 bases.

As disclosed herein, 100% sequence identity between the inhibiting nucleotide construct and the target endogenous gene is not required, and is virtually impossible to obtain in the event that the target viral sequence has changed due to genomic variation of the virus, to practice the present invention. Thus the invention has the advantage of being able to tolerate sequence variations that might be expected due to genetic mutation, strain polymorphism or evolutionary divergence.

For transcription from an expression DNA construct (for transgenic *in vivo* transcription), a regulatory region, such as a promoter, enhancer, splice donor and acceptor, or polyadenylation) may be used to transcribe the DNA. The RNA strand(s) may or may not be polyadenylated; the RNA strand(s) may or may not be capable of being translated into a polypeptide by the cell's translational apparatus. The use and production of an expression construct are known in the art (*vide* WO 97/32016; US 5,593,874, US 5,698,425, US 5,712,135, US 5,789,214 and US 5,804,693). Physical methods of introducing nucleic acids into cells and referred to as naked DNA transfer include injection of a solution containing the RNA or DNA, soaking the cells in a solution containing the RNA or DNA, or electroporation of cell membranes in the presence of the RNA or DNA. Other methods known in the art for naked DNA transfer may be used, such as lipid-mediated carrier transport, chemical-mediated transport, such as calcium phosphate and the like. Through naked DNA transfer, it is possible to insert the transgenic nucleotide sequence on a specific place in the genome of the target cell. This is done by engineering the transgenic nucleotide sequence in between parts which are homologous to, preferably non-coding, human sequences. This nucleotide sequence will then be inserted on a specific place in the genome through homologous recombination. Using this mechanism it is possible to insert the nucleotide sequence at a spot which is highly transcribed and where it does not disturb normal cell functions. The larger the length and the homology of the flanking sequences the higher the efficiency of site directed homologous recombination.

In order to avoid non-homologous recombination it is possible to flank the transgene cassette with thymidine kinase (tk) genes from *Herpes simplex.* Integration of (one of) the tk genes, which would be caused by a non-homologous integration, confers gangcyclovir sensitivity to the cell. Selection with gangcyclovir and a further selection agent (for which the resistance gene is present in the transgene cassette, e.g. the neomycin phospho-transferase gene) would then only yield cells with a transgene cassette at the desired locus.

A preferred method to introduce the nucleotide construct in the target cell is use of a viral vector system. The nucleotide construct is engineered into a viral vector DNA molecule, which packaged into a viral particle accomplishes efficient introduction of the nucleotide construct into the target cell. Most preferred are the use of viral vectors derived from adeno-associated virus (AAV), retroviruses, lentiviruses or adenovirus-AAV hybrid vectors. The methods for constructing such viral vectors and packaging into the viral particle are well known to a person skilled in the art.

The target cells to be transformed or transfected are preferably those cells which are the subject of viral infection. Of course, the virus which is used to transfect these cells must be able to infect these cells. AAV type 2 is known to infect hepatocytes and thus would be suitable to transfect liver cells *in vivo.*

As an alternative stem cells which generate the cells which are the target for viral attack could be used for transfection. In the case of HIV hemopoietic or lymphoid stem cells would be suitable.

Pluripotent hemopoietic stem cells are present in the hemopoietic tissues within the bone marrow of infants and adults. Approximately one out of 2000 cells is a stem cell. The stem cells divide and differentiate into either myeloid stem cells or lymphoid stem cells and from there into mature blood and immune system cells.

The myeloid stem cells are the precursors of granulocytes, mast cells, dendritic cells, monocytes, macrophages, erythrocytes and megakaryocytes. The erythrocytes become red blood cells and the megakaryocytes turn into platelets. All together the myeloid stem cells form the blood tissue and some cell types play a role in immunity. For example, the monocytes and macrophages play key roles in phagocytosis and antigen-presentation to lymphocytes. Granulocytes also show phagocytosis (neutrophils, eosinophils) or they release antimicrobial compounds (basophils). Mast cells also release pharmacologically active compounds at the site of infection. Dendritic cells play a key role in antigen presentation to T lymphocytes.

The lymphoid stem cells differentiate into B-lymphocytes in the bone marrow. The B-lymphocytes are the antibody producing cells and responsible for the humoral defence responses. In the bone marrow B-cells undergo an orderly sequence of immunoglobulin gene arrangements, which are antigen-independent. Mature naïve B-cells expressing membrane-bound IgM and IgD molecules, leave the bone marrow and migrate to the secondary lymphoid organs. Interaction with antigen, either directly or via antigen-presenting cells induces clonal selection of the affected B-cells in a process mediated by specific cytokines, secreted by activated T-lymphocytes (effector cells). B-cells divide and differentiate, generating populations of short-living plasma cells or long-living memory cells, which secrete specific immunoglobulins.

Lymphoid stem cells also migrate from the bone marrow to the thymus, where they differentiate into T-lymphocytes and (natural killer) NK-lymphocytes. From the thymus mature T-cells and NK-cells are actively dispersed to the peripheral tissues. The T- & NK-lymphocytes are responsible for the cellular defence responses. T-cells recognize antigens, which must be presented together with MHC molecules on the surface of antigen-presenting cells, cancer cells, virus-infected cells or grafts.

A sub-population of T-cells bearing CD4 recognize antigen associated with MHC class II molecules and generally function as T helper cells. Activation of T helper cells leads to cell division and gives rise to clones of effector cells. The effector cells secrete specific sets of cytokines, which play a central role in the activation of cells involved in specific responses, yielding memory, like mature B-cells or T cytotoxic cells and other cells involved in non-specific responses like NK cells, monocytes an macrophages.

T-cells bearing CD8 recognize antigen associated with MHC class I molecules and function as T cytotoxic cells. After antigen-binding by T cytotoxic cells, cytokines like IL-2 produced by T helper cells induce proliferation and differentiation of the T cytotoxic cells into cytotoxic T-lymphocytes (CTL's) and memory T cytotoxic cells, which are responsible for degradation of cells, which bear the respective antigens.

Thus, when using hemopoietic stem cells for transformation with a nucleotide construct according to the invention all the cells which are derived from said stem cell will bear the nucleotide construct in their genome and these cells will cause a blockade in the HIV replication and in further spread of HIV particles. Also they will not be killed by virus particle outburst and thus no AIDS symptoms will emerge. With an average lifetime of 100 days for a mature white blood cell, it will be easy to see that within a relatively short period of time all the infected and unprotected cells will disappear from the bloodstream, while they are replaced by cells which have been formed from the transgenic stem cells. If in these transgenic cells a provirus becomes active and starts to produce the gene products from transcription of the proviral DNA, the nucleotide construct of the invention, which is able to produce one transcript or multiple transcripts capable to fold into dsRNA will induce an intracellular and cytoplasmic mechanism in which homologous viral RNA molecules are specifically degraded, in a process referred to as RNA silencing. As a consequence, the expression of one or multiple viral genes, essential for virus replication, will be inhibited and no reproduction of virus particles and an eventual outburst of particles will occur anymore. Furthermore, the presence of the nucleotide construct of the invention in these cells does not impair the normal function of the cell.

For treatment of KSHV and EBV, which both occur in their latent forms in B lymphocytes also pluripotent hemopoietic stem cells can be transformed with a construct which expresses a double-stranded RNA which is able to inhibit replication of said viruses.

For other chronic viruses such as HCV, the target cells represent hepatocytes or alternatively, pluripotent hepatocytes. *Hepatitis* viruses infect and become resident in cells of the liver. Some of them, such as *Hepatitis* G and possibly also partly *Hepatitis* C appear to also reside in a latent state in blood cells. It is of course possible to treat liver stem cells (pluripotent hepatocytes) in a similar way as described herein for pluripotent hemopoietic and lymphoid stem cells.

Transformation of human hemopoietic stem cells is known in the art. Chatterjee *et al.* (Blood, 93: 1882-1894, 1999) describe a system for transduction of human myeloid progenitor cells using AAV. AAV is a single-stranded, replication-defective nonpathogenic human parvovirus with a 4.7 kb DNA genome with palindromic inverted repeats. Wild-type AAV requires coinfection with a helper virus such as adenovirus or herpes simplex virus for successful infection. In the absence of helper-virus coinfection, AAV integrates site-specifically and in a stable fashion, via the inverted repeats into the AAVS1 site of human chromosome 19. AAV vectors lacking one or multiple AAV genes integrate into other chromosomal sites than AAVS1. AAV vectors have high transduction frequencies in cells of diverse lineages and allow efficient transgene expression from RNA polymerase II- and III-dependent promoters. The results from Chatterjee *et al.* show that primitive myelo-erythroid progenitor cells are amenable to genetic modification by AAV vectors.

The target gene(s) of the virus which are subject for inhibition by the nucleotide construct of the invention are preferably genes that are essential for replication of the virus. In the case of HIV such genes are the *pol, tat* or *rev* genes. The *pol* gene is essential for virus infection and for the production of infectious virus particles. The *tat* and *rev* regulatory genes, with overlapping coding domains, are essential for provirus activation and accumulation of viral structural proteins and genomic RNAs. The sequences in the nucleotide construct can be derived from a single HIV gene or from multiple HIV genes. In the case of HCV, which only has a single gene, the target sequence can be any part of the viral genome.

To achieve production of a single transcript or multiple transcripts capable to fold into dsRNA (also called a hairpin structure or panhandle) a nucleotide DNA construct is made (the transgene cassette) harbouring both a sense and an antisense nucleotide sequence in opposite polarities (i.e. wherein the antisense sequence is an inverted repeat of the sense sequence) of at least 40 basepairs of which one is complementary to the target viral gene. A length of 40 nucleotides has been described to be minimally required for the production of an effective dsRNA which is able to block the target gene expression. Preferably larger stretches of DNA should be used, with a length of 100, 150, 200, 250, 300, 350, 400, 450 or 500 nucleotides. The sense and antisense nucleotide sequences can be connected through a spacer nucleotide sequence of any length (such as an intron sequence). If an intact intron sequence is used as spacer this intron may be removed during mRNA formation by endogenous enzymatic processes in the nucleus of the cell. The order of the sense and antisense sequence is not important. It is also possible to combine more than one sense-antisense combination in one and the same construct. The simple form can be depicted as: prom - S - spac - AS - term, wherein prom represents a promoter, S the target viral DNA sequence, AS the target viral DNA sequence in opposite polarity compared to S, spac a spacer sequence and term the transcriptional terminator DNA sequence. Also the following constructs can be applied: prom - S1 - spac - AS1 - spac - S2 - spac - AS2 - term, or prom - S2 - spac - S1 - spac - AS1 - spac - AS2 - term. Variations in the composition of the construct are possible, as long as the transcripts of said constructs may fold partially or fully into dsRNA. Alternatively, the nucleotide construct may consist of two separate transgene cassettes in which the target viral DNA sequence is present in opposite polarities towards the promoter(s). In short notation these constructs then look like: prom1-S-term1 and prom2-AS-term2. Prom1 and prom2 can be the same or different promoters, term1 and term2 can be the same or different terminators. Both constructs can be introduced into the cell on the same vector, but can also be introduced using two different vectors.

Alternatively, the nucleotide construct may consist of a single target viral DNA sequence flanked by promoters in opposite polarities in terms of transcriptional directions. In short notation such a construct then is formed by: prom1-S-prom2. Prom1 and prom2 can be the same or different promoters.

The transcriptional promoter is preferably derived from DNA viruses, including 5'-long terminal repeats from retroviruses and lentiviruses, the SV40 large T antigen promoter, the cytomegalus virus (CMV) immediate early promoter, and the like. These promoters and various others are easily obtainable for a person skilled in the art. As terminator any terminator applicable in human or animal cells can be used.

For an effective anti-HIV therapy basically the following steps should be taken:

### 1. isolation of stem cells

In the case of isolation of human hemopoietic stem cells from HIV-infected persons bone marrow punctions will be taken from HIV seropositive patients. These punctions generally harbour 10⁸ cells per millilitre, from which 50,000 are hemopoietic stem cells. Using standard enrichment techniques, 5 x 10⁵ CD34⁺38⁻ cells will be isolated per millilitre punction and in principle one out often of these cells is a HSC.

### 2a. construction of the transgenic cassette

According to generally used genetic engineering techniques a nucleotide construct will be made which comprises one or more promoters and terminators to enable transcription and formation of dsRNA as described above. Further, this expression product may comprise sequences necessary for homologous recombination flanked by the *Herpes simplex* tk genes. On the cassette preferably also a selectable marker (preferably an antibiotic resistance gene) is provided under separate transcriptional control, which would allow selection of transformed or transfected cells.

### 2b. transformation of stem cells

The cell fractions enriched for hemopoietic stem cells and lymphoid stem cells will preferably be transformed using homologous recombination. For this the nucleotide constructs as prepared according to the description above are inserted in a viral vector or are applied as 'naked DNA'. Transformation or transfection of stem cells is done using methods known in the art (e.g. Halene & Kohn, Human Gene Therapy 11: 1259-1267, 2000; Buchsacher & Wong-Stall, Blood 95: 2499-2504, 2000; Buchsacher & Wong-Staal, Human Gene Therapy 12: 1013-1019, 2001). After transformation cells harbouring the transgene cassette may be selected using a selection agent (antibiotic).

### 3. Reintroduction

The transformed HIV resistant hemopoietic stem cells and lymphoid stem cells will be re-introduced into the bone marrow of the patient and a new HIV resistant T helper cell population will be established protecting the patient from AIDS symptom development and rendering the patient non-infectious.

A person skilled in the art will be able to use the invention in similar set-ups for treatment of infections with other chronic viruses.

Furthermore, the invention can not only be used as a treatment for animals or humans which are already infected by a chronic virus but also prohylacticly to inhibit infection with such a virus. This would be especially recommendable for people who run a high risk of being infected with a chronic virus.

## Claims

1. A method for the inhibition of replication of a chronic virus in cells of an animal or human by introducing into target cells of the virus a nucleotide construct which is able to produce a single transcript or multiple transcripts capable of folding into double-stranded RNA with nucleotide sequence homology to one or more nucleotide sequences of the virus which are essential for replication of the virus.

2. A method according to claim 1 wherein a stem cell is provided with the nucleotide construct and wherein said stem cell is capable of generating the target cell of the virus.

3. A method for inhibition of HIV replication comprising
a. isolating hemopoietic or lymphoid stem cells from a person;
b. introducing into these stem cells a nucleotide construct which is able to produce a single transcript or multiple transcripts capable of folding into double-stranded RNA with nucleotide sequence homology to one or more nucleotide sequences of the virus which are essential for replication; and
c. re-introducing the stem cells in the person from whom they have been isolated.

4. Animal or human cell which is the target cell for a chronic virus, **characterized in that** it is transformed or transfected with a nucleotide construct which is able to produce a single transcript or multiple transcripts capable of folding into double-stranded RNA with nucleotide sequence homology to one or more nucleotide sequences of the virus which are essential for replication.

5. Animal or human stem cell which is able to generate a target cell for a chronic virus, **characterized in that** it is transformed or transfected with a nucleotide construct which is able to produce a single transcript or multiple transcripts capable of folding into double-stranded RNA with nucleotide sequence homology to one or more nucleotide sequences of the virus which are essential for replication.

6. Human hemopoietic or lymphoid stem cell, **characterized in that** it is transformed or transfected with a nucleotide construct which is able to produce a double-stranded RNA which is homologous to one or more nucleotide sequences of the human immunodeficiency virus which are essential for replication.

7. Nucleotide construct harbouring a nucleotide sequence of at least 40 nucleotides, which is homologous to a gene or part of a gene of a chronic virus which is able to infect an animal or human, wherein said nucleotide sequence is also present as an inverted repeat or wherein said nucleotide sequence is flanked by two promoters and which nucleotide construct, when transcribed yields one transcript or multiple transcripts capable of folding into a double-stranded RNA from said sequence and its inverted repeat, or from said sequence flanked by two promoters.

8. Nucleotide construct according to claim 7, wherein the nucleotide sequence and its inverted repeat are separated by an intron.

9. Nucleotide construct according to claim 7 or 8, **characterised in that** it comprises multiple nucleotide sequences of at least 40 nucleotides in length which show nucleotide sequence homology to different parts of the same gene or to different genes or parts thereof of said virus, wherein said nucleotide construct is also present as an inverted repeat, or wherein said nucleotide construct is flanked by two promoters .
